# EUROPEAN PATENT SPECIFICATION

(11) **EP 4 344 683 B1**
(45) Date of publication and mention of the grant of the patent: **08.04.2026**
(21) Application number: 23201040.5
(22) Date of filing: 29.09.2023
(51) Int. Cl.: A61F 2/66, A61F 2/68

(54) **ELASTIC ENERGY STORING FOR PROSTHESIS OR ORTHOSIS**
ELASTISCHE ENERGIESPEICHERUNG FÜR PROTHESE ODER ORTHESE
STOCKAGE D'ÉNERGIE ÉLASTIQUE POUR PROTHÈSE OU ORTHÈSE

(30) Priority: 30.09.2022 EP 22199286
(43) Date of publication of application: 03.04.2024
(73) Proprietor: Université catholique de Louvain, 1348 Louvain-la-Neuve (BE)
(72) Inventor: HEREMANS, François, 1348 Louvain-la-Neuve (BE); RONSSE, Renaud, 1348 Louvain-la-Neuve (BE); EVRARD, Jeanne, 1348 Louvain-la-Neuve (BE)
(74) Representative: Icosa

(56) References cited:
- US-A1- 2015 018 975
- US-A1- 2017 165 088
- US-A1- 2021 307 938
- US-A1- 2022 142 793

## Description

### FIELD OF INVENTION

The present invention relates to the field of prosthesis or orthosis. More precisely, the present invention relates to the field of elastic energy storing and releasing for use in a prosthesis or orthosis.

### BACKGROUND OF INVENTION

Many types of prostheses or orthoses exist, which can be divided into passive and active devices. Existing active lower-limb prostheses or orthoses have demonstrated their ability to supply the net positive energy being required during flat ground walking, and more complex tasks such as slope and stair ascend, which is not possible with passive devices. However, the added-value of active devices is significantly impacted by their limited energetic autonomy and excessive weight.

In an effort to reduce weight and encumbrance, existing active prostheses or orthoses embed series elastic actuators (SEA), corresponding to serial connections of an actuator and a spring which, if correctly tuned, have a direct effect in decreasing the motor speed and thus decrease the required peak mechanical power such as described for example in the US patent application US 2021/307938. This offers to equip the prostheses or orthoses with smaller motors than those necessary to provide the whole peak power. In addition to series elastic actuators, it has been proposed to embed a parallel spring passively generating torque in order to reduce the torque demand of the actuator. The motor torque is proportional to its current, and the motor Joule losses are proportional to the square of this current. Consequently, the torque directly influences the motor dimensioning, and thus its cost, weight, and potential hazard for the user. Moreover, the motor output torque also impacts the mechanical transmission (*i.e.,* gear ratio) and thus the reflected inertia and back drivability of the device. With a parallel spring, the actuator produces only the remaining fraction of the whole requested joint torque.

In existing active prostheses or orthoses for gait assistance, the parallel spring is implemented in two different ways, depending on the joint angle where torque production is triggered. The first type engages above a fixed angular threshold in order to not impede with the joint motion during the swing phase such as in the US patent application US 2022/142793. However, in this case, the parallel spring only provides a reduced fraction of the total elastic response. Moreover, the prosthesis or orthosis cannot adapt to different terrains, e.g. slopes, where the ideal joint kinematic would differ. The second type can dynamically change the angle of engagement. Engaging early in the stance phase allows to store more elastic energy but requires the parallel spring to be deactivated during the swing phase. However, to date, such adaptive mechanisms rely on complex clutch being coaxial with the joint rotation axis, resulting in complex and bulky prosthetic or orthotic devices.

Finally, in few existing active protheses or orthoses, the storage of the energy by the springs is performed thanks to a locking mechanism allowing the application of a force for the extension or compression of the spring only during part of the gait cycle such as in the US patent application US 2017/0165088. When the locking mechanism is locked, the energy stored in the spring is stored and then released while the energy storage is impossible when the locking mechanism is unlocked. Most of the locking mechanisms are based on at least one gear wheel such as a ratchet which is locked by a pawl that engages the teeth such as in the US patent application US 2015/0018975. However, the use of a gear wheel implies the locking of the spring only in a discrete number of joint positions determined by the positions of the gear wheel and the pawl. Moreover, the sudden locking of the mechanism may lead to discomfort for the user during the walk.

Thus, there is a need for an active prosthesis or orthosis which achieves high mechanical performance while having reduced overall power consumption, the prosthesis or orthosis additionally being lightweight, robust and exhibiting a simple, compact and adjustable structure or allowing a soft and comfortable use.

A purpose of this invention is therefore to provide an active prosthesis or orthosis comprising a unidirectional locking system, a slider and a priming system configured to switch between a first state of the device allowing a free movement of the device and a second state of the device allowing to store mechanical energy developed by the user's body. The absence of gear wheel allows a soft locking and infinite - or continuous - locking positions of the mechanism thereby improving the comfort of the user. Moreover, the low number of elements provides a simple and lightweight structure. Finally, the invention requires no energy during most of the loading cycle, for example the gait cycle. An actuator may be provided if additional impulse needs to be provided to the user.

### SUMMARY

To this end, the present invention relates to a device for storing mechanical energy comprising:
- A support comprising a front side and a rear side defining a curvilinear axis;
- A carriage connected to at least one elastic element, each elastic element being configured to store energy and one extremity of each elastic element being connected to the support;
- A unidirectional locking system;
- A slider slidably inserted into the carriage;
- A priming system configured to switch between a first state of the device and a second state of the device and vice-versa;

wherein the carriage is configured to move, the move of the carriage leading to storage of energy by the at least one elastic element,
wherein the slider is configured to slide inside the carriage,
wherein, in the first state, the unidirectional locking system is disabled,
wherein, in the second state, the unidirectional locking system is primed, the slider is free to slide in a first direction into the carriage and the move of the slider in the reverse direction into the carriage is prevented.

Indeed, the device allows an instantaneous blocking of the slider at any position leading to a soft locking of the mechanism thereby improving the comfort of the user.

Furthermore, the device may be bistable implying a low consumption during the use of the device since each stable state requires no energy to be maintained. Moreover, this implies a weight reduction of the device thanks to the reduced size of the battery.

According to other advantageous aspects of the invention, the carriage is configured to move along the curvilinear axis, the move of the carriage backwards leading to storage of energy by the at least one elastic element, the slider being configured to slide inside the carriage along the curvilinear axis.

According to other advantageous aspects of the invention, in the first state, the unidirectional locking system is abutted towards the front side of the support.

According to other advantageous aspects of the invention, the priming system comprises at least one engaging system fixed to the support or to the carriage and at least one plunger, the engaging system switching the plunger between a first state of the device and a second state of the device.

This allows to avoid, in the first state, any unwanted priming of the unidirectional locking system.

Moreover, the priming system allows to trigger the storage of mechanical energy by the device at different joint angles thereby increasing the reliability of the device.

According to other advantageous aspects of the invention, the at least one engaging system is an electromagnet and the at least one plunger is a magnet fixed to the unidirectional locking system, the device being in the first state when the electromagnet and the magnet are in contact and the device switching to the second state when the electromagnet is powered up.

In this bistable embodiment, the use of electromagnets allows to reduce the energy consumption of the device.

According to other advantageous aspects of the invention, the at least one engaging system is a stator coil and the at least one lockable plunger is a magnet slidably inserted into the stator coil, the device switching to the second state when the stator coil is powered up.

The advantage of this monostable embodiment is the ability to switch back automatically to the first state when the load applied on the device disappears. Moreover, the use of a stator and a magnet optimizes the force and power density of the priming system leading to a smaller and lighter device.

According to other advantageous aspects of the invention, the priming system further comprises at least one secondary elastic element and wherein, when the device is in the first state, each elastic element comprises a first potential energy and when the device is in the second state, each elastic element comprises a second potential energy smaller than the first potential energy.

The use of a secondary elastic element in the monostable embodiment allows reducing the energy consumption of the device because the switch from the second state to the first state is passively permitted by the energy stored in said secondary elastic element.

According to other advantageous aspects of the invention, the carriage comprises a cavity having a section decreasing in the backward direction, the slider and the unidirectional locking system being slidably inserted into the cavity and wherein, in the first state the unidirectional locking system is in the front side of the carriage and, in the second state, the unidirectional locking system is in the back side of the carriage.

This embodiment allows a simple and thus lightweight device permitting a soft locking mechanism for storing the energy. Moreover, the structure is adjustable to the user.

According to other advantageous aspects of the invention:
- The unidirectional locking system comprises rollers in a roller cage, and, in the second state:
   - the slider and the carriage are in contact with rollers; and
   - the slider, the rollers and the carriage are buttressed;
- The device comprises at least one sensor, the signal recorded by the at least one sensor triggering the switch from the first state to the second state; or
- The device comprises a controller controlling the priming system according to the signal recorded by the sensor.

According to other advantageous aspects of the invention, the device comprises an actuator configured to actively slide the slider towards the front side or the rear side of the support.

The actuator allows to provide additional energy to the system. Moreover, the actuator may help to place the device in the first state.

According to other advantageous aspects of the invention, the device comprises a ballscrew allowing to move the slider along the curvilinear direction, wherein the curvilinear direction is longitudinal; and wherein the actuator comprises a motor dragging a belt-pulley transmission thereby rotating the ballscrew.

The present invention also relates to a system comprising:
- a first body;
- a second body comprising the device;
- an articulated joint between the first body and the support of the second body, the articulated joint allowing the rotation of the first body and the support of the second body with respect to one another around a joint rotation axis;
- a transmission element between the articulated joint and the slider of the device; wherein the transmission element is configured so that, when the first body rotates around the articulated joint, mechanical energy is transmitted to the device through the transmission element as a force applied to the slider.

This system allows to efficiently store the energy provided by the rotation of the joint for a subsequent release.

According to other advantageous aspects of the invention, the system is a prosthesis or an orthosis for a joint.

This allows to provide a foot prosthesis allowing a person to perform a comfortable walk. Moreover, the low-energy consumption of the system allows the person to walk during long time.

### DEFINITIONS

In the present invention, the following terms have the following meanings:
**"Bistable"** refers to an embodiment in which the device has the capacity to switch between two passive stable states, the switching requiring a minimum of energy consumption.

"**Dorsiflexion**" refers to the flexion of the foot in an upward direction, i.e., the toes move towards the lower leg. In dorsiflexion, the ankle angle α between the lower leg and the foot increases.

**"Front side"** or **"forward direction"** refers to the moving direction of the carriage when releasing energy from the elastic element.

**"Monostable"** refers to an embodiment in which the device has only one passive stable state requiring no energy consumption.

**"Plantarflexion"** refers to the flexion of the foot in a downward direction, i.e., towards the sole. In plantarflexion, the ankle angle α between the lower leg and the foot decreases.

**"Rear side"** or **"backward direction"** refers to the moving direction of the carriage when storing energy in the elastic element.

### DETAILED DESCRIPTION

This invention relates to a device 100 for storing mechanical energy comprising:
- A support 101;
- A carriage 103 connected to at least one elastic element 104;
- A unidirectional locking system 105;
- A slider 106 slidably inserted into the carriage 103; and
- A priming system 107.

The device 100 may be used in active prostheses or orthoses. For example, the device 100 is used in a prosthesis or an orthosis for an articulated body member. The device 100 therefore allows to store the mechanical energy developed by the user's body towards the device 100 and to release it afterwards so that the prosthesis or orthosis performs a movement.

The device 100 may be a part of a prosthesis or an orthosis for an ankle, dedicated to locomotion assistance.

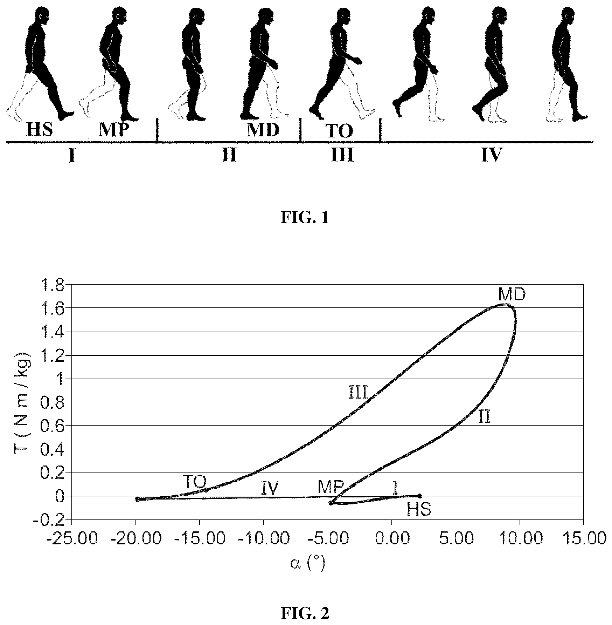

As illustrated in figure 1, the normal walking cycle of a person can be divided into four different stages, among which stages I to III correspond to the stance phase, where the foot is in contact with the ground, and stage IV corresponds to the swing phase, during which the foot is out of contact with the ground and the leg is swept forward in order to make the next step at the next heel-strike.

The first stage I extends from heel strike (HS) until the foot is completely on the ground, which corresponds to the first maximum plantarflexion (MP) following the heel strike. During this stage I, as represented in figure 2, the ankle joint is exerting a braking torque between the foot and the lower leg in order to prevent the foot from falling on the ground. Energy for exerting said torque is supplied by the person walking and the torque is directed in a direction such that the plantarflexion is braked.

The next stage II, called the stage of dorsiflexion, is the stage during which the lower leg is brought forward, i.e., the lower leg is turned towards the foot which is still on the ground, until maximum dorsiflexion MD. During this stage II, the walking person is again supplying energy, which is stored in the muscles and tendons, and the body is decelerated.

The next stage III is the stage during which the foot is pushed-off and is leaving the ground. In this stage III, the energy stored in the muscles and tendons during stage II is converted to motion energy by pushing off with the toe, until toe off TO, and the body is accelerated.

The last stage IV, corresponding to the swing phase, is the stage during which the foot is rotated around the ankle in order to bring the foot back in its original position at heel strike HS. During the swing phase, the foot is not in contact with the ground and almost no energy is required to rotate the foot.

In the embodiment of a prosthesis or an orthosis for an ankle, the device 100 of the invention stores the energy generated by the user's body during the stance to release it with high power during the push-off until the toe-off, *i.e.,* during the time range when only the toes are in contact with the ground, thereby providing the impulse to the user when it is the most efficient to initiate the swing phase and setting up the device 100 for the next step.

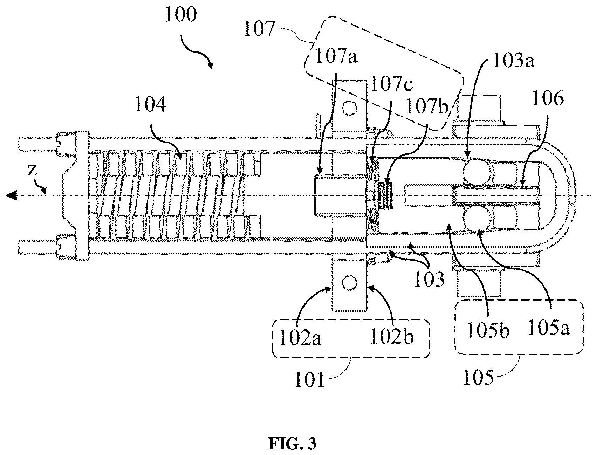

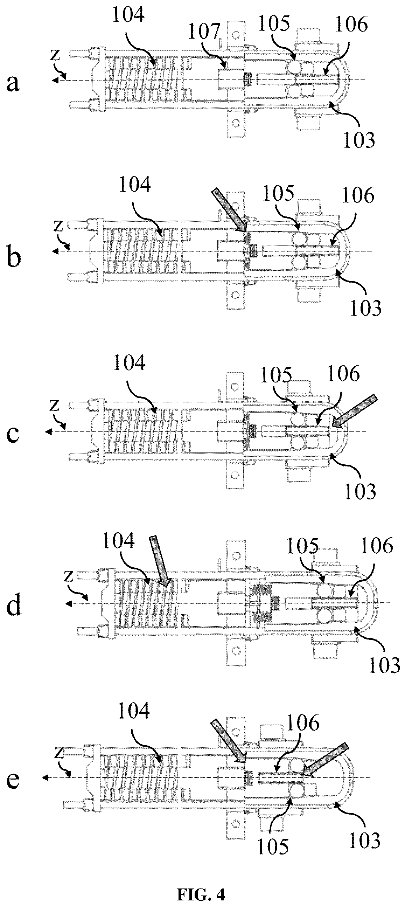

Hereafter, the different components of the device 100 will be detailed and their function will be described in the preferred embodiment of an ankle joint. The different components of the device 100 are represented in figure 3 while the configurations of the device 100 as a function of the stage of the gait are represented in figure 4.

### Carriage 103 and slider 106

The mechanical energy (for example generated by the body forward progression during the gait) is stored in each elastic element 104. To do so, one extremity of each elastic element 104 is connected to the support 101, the other extremity being connected to the carriage 103. For example, the elastic element 104 is a spring.

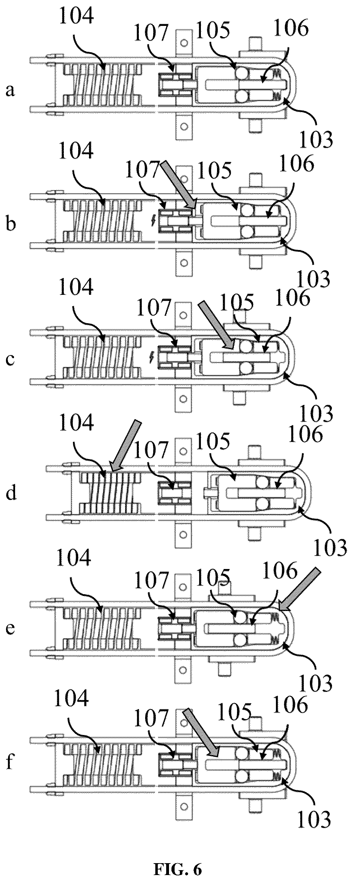

The carriage 103 is configured to move. For example, the carriage 103 is configured to move along a curvilinear axis z inside the support 101. Preferably, the curvilinear axis z is linear (as a straight line) as represented in figures 3, 4a-e and 6a-f. When the carriage 103 is moving towards one direction (for example the backward direction), each elastic element 104 stores mechanical energy (as represented in figures 4d and 6d). For example, the elastic elements 104 are compression springs, the elastic elements 104 being compressed or stretched by the motion of the carriage 103. Conversely, when each elastic element 104 returns on its resting state thereby releasing the stored mechanical energy, the carriage 103 is moving towards the other direction defined as the forward direction (as represented in figures 4e and 6e). In figures 3, 4a-e and 6a-f, the curvilinear axis z is represented by the dashed arrow pointing towards the forward direction. In the example of an ankle joint, the backward direction is preferably pointing to the heel while the forward direction is preferably pointing to the toes.

These directions allow to define the front side 102a and rear side 102b of the support 101. The front side 102a is the side towards which the carriage 103 goes when moving in the forward direction. Conversely, the rear side 102b is the side towards which the carriage 103 goes when moving in the backward direction. The front side and rear side define the curvilinear axis z. Preferably, the curvilinear axis z is longitudinal, *i.e.,* in the general longitudinal length of the device 100.

As represented in figures 3, 4a-e, 5 and 6a-f, the carriage 103 may comprise a cavity 103a having a section decreasing in the backward direction. In other words, the contact planes are sloped so that they can initiate a buttressing (overarching) locking. The slider 106 and the unidirectional locking system 105, detailed hereafter, are slidably inserted into the cavity 103a.

### Priming system 107

The priming system 107 is configured to switch between a first state and a second state of the device 100 and vice-versa. The first and second states are not necessarily passive states. Indeed, they may require energy to be maintained.

In a bistable embodiment of the device 100, the first and second states may be stable states, *i.e.,* requiring no energy to be maintained.

In a monostable embodiment of the device 100, the first state does not require energy to be maintained while the second state requires energy to be maintained.

In the first state (represented in figures 4a, 4e, 6a and 6e-f), the unidirectional locking system 105 is disabled. In this state, the unidirectional locking system 105 is preferably abutted towards the front side 102a of the support 101. Moreover, the slider 106 is free to move into the carriage 103. Preferably, the slider 106 is free to move into the carriage 103 along the curvilinear axis z in a first and a second direction (reverse from the first direction). In this state, the carriage 103 may also abut towards the front side 102a of the support 101. In the bistable embodiment and for an ankle joint, the first state is achieved when the joint reaches the minimum ankle angle α during the swing phase. In the monostable embodiment and for an ankle joint, the priming system 107 automatically switches to the first state when the load disappears (during the push-off).

In the second state (represented in figures 4b-d and 6b-d), the unidirectional locking system 105 is primed. The priming of the unidirectional locking system 105 allows the slider 106 to move freely along the curvilinear axis z in the first direction into the carriage 103 (as represented in figure 4c) and prevents the slider 106 to move in the second direction. Therefore, when an external force is applied to the slider 106 in the second direction, the movement of the slider 106 is totally transferred to the carriage 103 configured to move along the curvilinear axis z inside the support 101 so that it moves in the backward direction (as represented in figure 4d). In the example of an ankle joint, the second state is reached after the end of the swing phase and before the maximum plantarflexion (MP). In the bistable embodiment and for an ankle joint, the second state can be achieved at any time between the end of the swing and heel strike. In the monostable embodiment and for an ankle joint, the unidirectional locking system 105 is primed at any time between the heel strike and the maximum plantarflexion (MP). The external force allowing to store energy in the at least one elastic element 104 is therefore generated by the movement of the body-weight in the forward direction.

The first and second directions are relative to the carriage 103 *(i.e.,* are defined in a coordinate system fixed to the carriage 103). For example, the carriage 103 may be in movement towards the forward direction with a first velocity while the slider 106 slides in the forward direction with a second velocity larger than the first velocity. In this example, the slider 106 moves towards the front side 102a relatively to the support 101 and also towards the front side 102a relatively to the carriage 103. Another example is when the device 100 is in the second state and when the energy stored in the at least one elastic element 104 is released. The carriage 103 is moving towards the forward direction taking the slider 106 with it since the movement of the slider 106 in the backward direction is prevented. The slider 106 is thus resting relatively to the carriage 103 but in movement towards the forward direction relatively to the support 101. In other words, the first and second directions are not absolute direction contrarily to the forward and backward directions.

When the carriage 103 comprises a cavity 103a having a section decreasing in the backward direction, the priming of the unidirectional locking system 105 implies a movement of said system 105 towards the back side of the carriage 103. Advantageously, this configuration allows a soft locking mechanism and infinite number of locking positions. Indeed, thanks to the decreasing section of the cavity 103a, in the second configuration, the unidirectional locking system 105 is comprised in a section of the carriage 103 which is small enough to block the slider 106 by buttressing the unidirectional locking system 105 between the slider 106 and the carriage 103 thus preventing the slider 106 to move in the backward direction. In other words, the locking action is generated by a buttressing situation against two non-parallel surfaces. By choosing an appropriate relative angle between the surfaces, when buttressing, any longitudinal motion increases the contact forces until equilibrium is reached. Since the decreasing section is progressive, the blocking of the slider 106 is performed instantaneously at any position (i.e., not at discrete joint positions) thereby increasing the comfort of the user compared to systems comprising gear wheel and/or ratchet wheel.

The priming system 107 may comprise at least one engaging system 107a fixed either to the support 101 or to the carriage 103 and at least one plunger 107b.

The priming system 107 may further comprise at least one secondary elastic element 107c. For example, the at least one secondary elastic element 107c is a spring.

In an example of bistable embodiment, the at least one engaging system 107a is an electromagnet and the at least one plunger 107b is a magnet fixed to the unidirectional locking system 105. In this example, the electromagnet comprises a magnetic circuit and a coil. When the magnet of the unidirectional locking system 105 is close enough to the electromagnet, a magnetic flux is created inside this magnetic circuit thereby attracting the magnet. When the device 100 is in the first state, the electromagnet and magnet are in contact by the magnetic flux attraction. The unidirectional locking system 105 is thus in the front side of the carriage 103 so that it is disabled. This attraction prevents the unidirectional locking system 105 from moving and thus prevents locking of the slider 106. In the embodiment wherein priming system 107 comprises at least one secondary elastic element 107c, said element 107c is calibrated so that, when the magnet is in contact with the electromagnet (first state), a predetermined quantity of energy is stored in the at least one secondary elastic element 107c and the force generated but the secondary elements 107c is lower than the attraction force between the magnet and the electromagnet. In the embodiment wherein the electromagnet is fixed to the support 101, the generated force should also be lower than the one generated by elastic deformations of the at least one elastic element 104. The electromagnet may be briefly powered up so that a second magnetic flux is created which cancels the magnetic flux of the magnetic circuit. The resulting (total) magnetic flux is almost zero. Therefore, when the electromagnet is briefly powered up (switching to the second state), the unidirectional locking system 105 is slightly moved towards the backward direction by releasing the predetermined quantity of energy stored in the at least one secondary elastic element 107c. For example, each elastic element 107c is a spring with a first end fixed to the front side 102a of the support 101 or the carriage 103 and a second end configured to be in contact with or fixed on the unidirectional locking system 105 in the first state. In this embodiment, the first and second states are a passive state since they do not require energy to be maintained. This embodiment is advantageous because energy is only required to power up the electromagnet allowing to switch from the first to the second state. In the embodiment wherein priming system 107 does not comprise any secondary elastic element 107c, the electromagnet may be powered up in the second state so that a second magnetic flux is created leading to a magnetic repulsion between the electromagnet and the magnet. Therefore, when the electromagnet is powered up, the unidirectional locking system 105 is slightly moved towards the backward direction (primed). The electromagnet should remain powered up during at least part of the duration during which the device 100 is in the second state so that the unidirectional locking system 105 remains in the rear side of the support 101. In the second state, the unidirectional locking system 105 is thus not abutted towards the front side 102a of the support 101 but the stiffness of the at least one elastic element 104 may be high enough to keep the carriage 103 abutted to the front side 102a of the support 101 as long as mechanical energy is not stored in the at least one elastic element 104. In this embodiment, the first state is a passive state but the second state requires energy to be maintained.

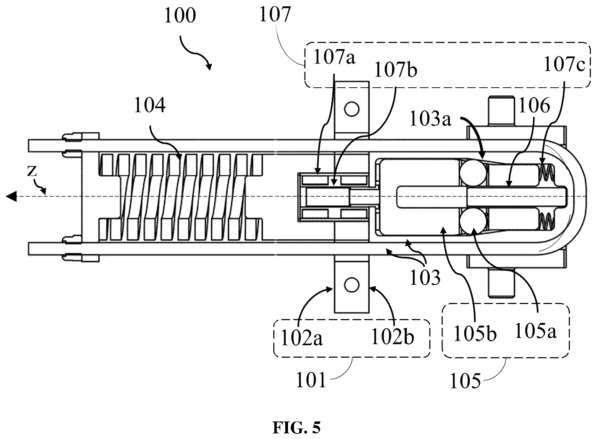

In an example of monostable embodiment, at least one engaging system 107a is a stator coil and the at least one plunger 107b is a magnet slidably inserted into the stator coil, as represented in figures 5 and 6. Preferably, the central axis of the stator coil is parallel to the curvilinear axis z. The magnet may be fixed to the unidirectional locking system 105. In this embodiment, the slide of the magnet inside the stator coil thus implies a slide of the unidirectional locking system 105 in the same direction. In this monostable embodiment, the stator coil is powered up, so that it creates a magnetic force sliding the magnet towards the rear side 102b leading to a switch between the first and the second state. The stator coil remains powered up during at least part of the duration during which the device 100 is in the second state. Therefore, in the second state, the power up of the stator coil leads to the priming of the unidirectional locking system 105 as represented in figures 6b-6c. In the first state (figure 6a), the stator coil is not powered up. Therefore, the magnet is free to slide in both directions in the stator coil. In this monostable embodiment, the first state is a passive state since it does not require energy to be maintained. On the contrary, the second state is an active state requiring energy to power the stator coil. This monostable embodiment is advantageous because the stator coil may automatically be powered on at a predetermined stage of the gait cycle. For example, when used in an ankle joint, the stator coil may automatically be powered on at the heel strike thereby triggering the storage of mechanical energy in the elastic elements 104 at different ankle angles α between the lower leg and the foot, then the stator coil may automatically be powered off when the elastic elements 104 are loaded.

Still considering the monostable embodiment, in the embodiment wherein the priming system comprises at least one secondary elastic element 107c, said element 107c is configured so that, when the device 100 is in the second state, each secondary elastic element 107c comprises a potential energy and when the device 100 is in the first state, each secondary elastic element 107c is in its resting state (with a lower potential energy) and the unidirectional locking system 105 is abutting towards the front end 102a of the carriage 103 as represented in figure 6a. For example, each elastic element 107c is a compression spring with a first end fixed to the rear side 102b of the carriage 103 and a second end configured to be fixed to the unidirectional locking system 105. Preferably, the potential energy is lower than the energy that can be produced by the stator coil. In the embodiment wherein the stator coil is fixed to the support 101, the potential energy should also be lower than the potential energy stored in the at least one elastic element 104. For example, the total potential energy of the at least one elastic element 107c is lower than a tenth, preferably a hundredth even more preferably a thousandth of the potential energy that may be stored in the at least one elastic element 104. Therefore, when the device 100 is switching to the second state, the unidirectional locking system 105 is primed and the carriage 103 is still abutting towards the front end of the support 101 (figure 6c). When the device 100 is storing energy in the elastic element 104, the stator coil may be powered off because the slider 106, the carriage 103 and the unidirectional locking system 105 are buttressed and the slider 106 is slid towards the backward direction thereby preventing the disabling of the unidirectional locking system 105 despite the force exerted by the secondary elastic element 107c (figure 6d). When the stator coil is powered off and the slider 106 slides in the first direction relatively to the carriage 103, energy stored in the at least one spring is released. Consequently, the unidirectional locking system 105 is abutting against the support 101 (front side of the carriage 103) as represented in figure 6f and the device 100 is automatically set-up for the next step.

In both embodiments of the priming system 107 - bistable and monostable - the priming of the unidirectional locking system 105 is active. The two embodiments differ only on the quantity of energy required to maintain the locking. This thus leads to an active prothesis and orthosis.

### Unidirectional locking system 105

As explained above, the unidirectional locking system 105 allows blocking the slider 106 to prevent its sliding towards the backward direction when the device 100 is in the second state.

To do so, the unidirectional locking system 105 may comprise rollers 105a in a roller cage 105b. Therefore, in the second state, the slider 106 and the carriage 103 are in contact with rollers 105a since the roller cage 105b is pushing backwards on the rollers 105a to bring them in contact with the slider 106 and the carriage 103, for example thanks to the sloped surfaces of the carriage 103. Therefore, in the second state, the slider 106, the roller 105a and carriage 103 are buttressed when a force is applied to the slider 106 in the backward direction.

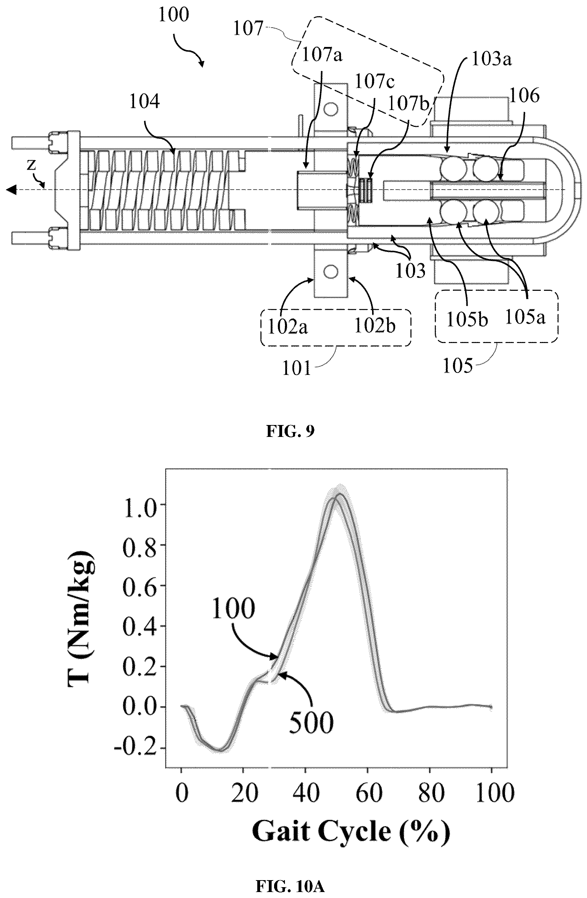

Preferably, the unidirectional locking system 105 comprises at least one pair of rollers 105a. When the unidirectional locking system 105 comprises more than one pair of rollers 105a, the rollers 105a, which preferably present a rotational axis, are disposed so that their rotational axes are not aligned. The rollers 105a are thus disposed side by side along the length of the slider 160. An embodiment wherein the unidirectional locking system 105 comprises two pairs of rollers 105a is illustratively represented in figure 9.

The buttressing configuration allows the transfer of longitudinal forces between the slider 106 and the carriage 103 as long as rolling of the rollers 105a is guaranteed. This last condition is maintained if the contact forces at the two contact points of each roller 105a remain within the friction cone of the materials of the surface of the slider 106, the carriage 103 and the rollers 105a. The advantage of using more than one pair of rollers 105a is that the contact forces are distributed over more contact areas, thus preventing a local wear of the elements.

For example, the surface of the rollers 105a, the surface of the carriage 103 and/or the surface of the slider 106 may be in plastic thereby reducing the weight of the device 100. In another example, the surface of the rollers 105a, the surface of the carriage 103 and/or the surface of the slider 106 may be unlubricated steel, preferably hardened steel. This is advantageous because steel provides durability to the device.

### Control elements of the device 100

The disabling or priming of the unidirectional locking system 105 by the priming system 107 may be controlled by the device 100 itself.

For example, the device 100 may further comprise at least one sensor 108. The signal recorded by the at least one sensor 108 triggers the switch from the first state to the second state. In the example of an ankle joint, at least one of the sensors 108 may be an accelerometer measuring the acceleration of the device 100 or of the foot, a joint position sensor, or a pressure sensor measuring the pressure undergone by the heel part of the device. For example, these sensors are able to determine the heel strike to trigger the priming of the unidirectional locking system 105 in the monostable embodiment. In another example, these sensors are able to determine the end of the swing phase to trigger the priming of the unidirectional locking system 105 in the bistable embodiment.

The device 100 may further comprise a controller 109 configured to control the priming system 107 according to the signal recorded by the sensor 108.

### Actuator 110

The device 100 may also comprise an actuator 110 configured to actively slide the slider 106 towards the front side 102a or the rear side 102b of the support 101.

For example, the actuator 110 may act as an additional source of energy and thus as a complement of the at least one elastic element 104. In the example of an ankle joint, the actuator 100 may be turned on during the push-off, *i.e.* during the high power time range before the toes leave the ground. The time range of the push-off may be determined, for example, thanks to the signal measured by the at least one sensor 108. When turned on, the actuator 110 slides the slider 106 towards the front side 102a of the support 101 thereby increasing the torque created by the device 100. This is advantageous because the actuator provides additional torque in complement to the one created by the at least one elastic element 104 during the push-off. Moreover, the use of the actuator 110 may further allow to switch the device 100 in the first state by abutting the unidirectional locking system 105 towards the front side 102a as represented in figure 4e.

In another example, the actuator 110 creates a force that brakes the forward progression of the slider 106. In the example of an ankle joint, the actuator 110 slides the slider 106 towards the rear side 102b of the support 101 during the stage I thereby exerting a braking torque between the foot and the lower leg in order to prevent the foot from falling on the ground as represented in figure 4a.

The actuator 110 may comprise a ballscrew 111. The ballscrew 111 is configured to move the slider 106 along the longitudinal curvilinear direction z. To do so, the actuator 110 further comprises a motor 110a dragging a belt-pulley transmission 110b thereby rotating the ballscrew 111.

### System 200

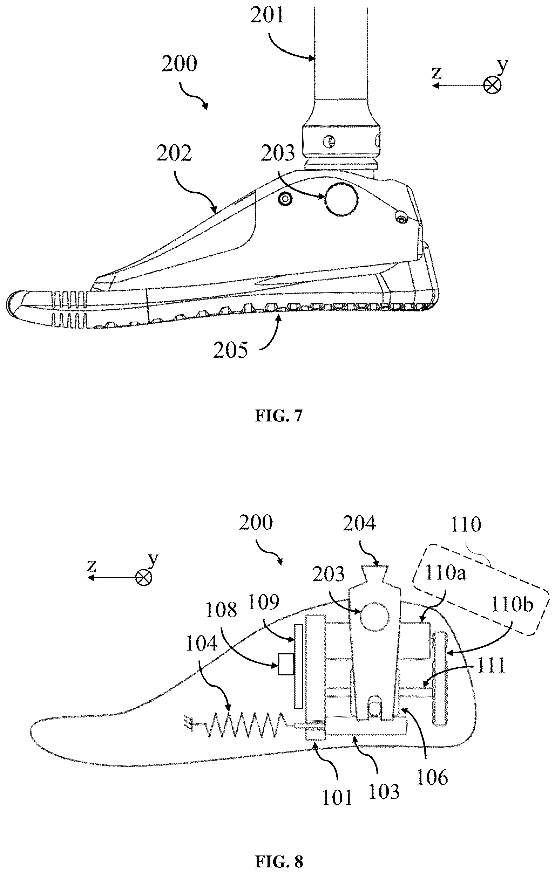

This invention also relates to a system 200, represented in figures 7 and 8, comprising:
- a first body 201;
- a second body 202 comprising the device 100;
- an articulated joint 203 between the first body 201 and the support 101 of the second body 202;
- a transmission element 204 between the articulated joint 203 and the slider 106 of the device 100;

The articulated joint 203 allows the rotation of the first body 201 with respect to the support 101 of the second body 202 around a joint rotation axis y. In the example of an ankle joint, the joint rotation axis y is preferably perpendicular to the linear direction z.

The transmission element 204 is configured so that, when the first body 201 rotates around the articulated joint 203, mechanical energy is transmitted to the device 100 through the transmission element 204 as a force applied to the slider 106 preferably along the curvilinear axis z.

Reversely, when the at least one elastic element 104 releases the stored energy, the released energy is transmitted to the first body 201 through the transmission element 204 by the move of the slider 106.

The system 200 may be a prosthesis or an orthosis for a joint such as ankle, knee, hip, wrist, elbow, shoulder. Preferably, the system 200 is a prosthesis or an orthosis for an ankle. In this preferred embodiment, the support 101 may be fixed to an insole 205. Advantageously, the insole 205 allows a protection of the device 100 while walking.

In another embodiment, the system 200 may be inserted in a prosthesis or an orthosis for a joint to control activation of some parts. For instance, a damper in a knee prosthesis could be activated or disabled according to the first and second states of the priming system 107. For instance, a mechanical transmission between a knee and an ankle could be activated or disabled according to the first and second states of the priming system 107. For instance, energy could be stored in a knee then transferred to an ankle - or vice versa - with two systems 200 in cooperation.

The system 200 may be also included in a more global exoskeleton, either for rehabilitation of subjects after an injury or for support of subjects in their activities.

The system 200 may be a component of a robotic system, allowing for energy storage or a change of configuration of a robot - configuration switched from one state to another based on the two states of the priming system 107.

In particular, the system 200 is a suitable part of a walking biped robot, with features disclosed hereabove, including energy storage, enabling or disabling of functions - damper, mechanical link... - and change of configurations.

The system 200 may be used also in other kind of robots, in which energy is stored slowly but released suddenly. This feature is especially interesting for hopping robots, or when a robot is not in the adequate position and should change - jumping, reversal, hopping...

Besides prosthesis and robotic domains, the system 200 may be included in industrial equipment, where an asymmetric activation is required, for instance in case of failure. An emergency brake could be activated - switching in second state of the priming system 107 upon power cut - to limit a movement for instance.

### Sub-system

The invention has been described hereabove in a context of energy storage with an elastic element 104. It can be noted however that the sub-system providing the asymmetric behavior of the device is of key importance.

Accordingly, an asymmetric device comprising a casing, a unidirectional locking system 105, a slider 106 slidably inserted into the casing and a priming system 107 - with the features and functional relationships disclosed hereabove, where the casing is analogous to the parts of the carriage 103 and support 101 in which the unidirectional locking system 105 is trapped - is another aspect of the disclosure.

Such an asymmetric device enables to control a mechanical link - including energy transfer or motion of parts - depending on the direction of movement of elements linked to the asymmetric device, or depending on the stage of a cyclic movement of elements linked to the asymmetric device.

Therefore, another object of this disclosure is an asymmetric device comprising:
- A casing;
- A unidirectional locking system;
- A slider slidably inserted into the casing;
- A priming system configured to switch between a first state of the asymmetric device and a second state of the asymmetric device and vice-versa;

wherein the slider is configured to slide inside the casing, preferably along a curvilinear axis,
wherein, in the first state, the unidirectional locking system is disabled and is abutted towards the front side of the casing,
wherein, in the second state, the unidirectional locking system is primed, the slider is free to slide in a first direction into the casing and the move of the slider in the reverse direction into the casing is prevented.

The asymmetric device may comprise all features disclosed hereabove in the detailed description and technically compatible.

The asymmetric device is preferably inserted in a mechanical system.

In particular, the casing of the asymmetric device may be a carriage moving relative to a support, the carriage being connected to at least one elastic element, each elastic element being configured to store energy and one extremity of each elastic element being connected to the support, and wherein the casing is configured to move along the curvilinear axis, thereby allowing storage of mechanical energy.

### BRIEF DESCRIPTION OF THE DRAWINGS

**Figure 1** is a representation of the different stages of the normal gait cycle.
**Figure 2** is a graph showing the evolution of the torque T experienced in the human ankle joint during the four different stages of the normal gait cycle, as a function of the ankle angle α between the lower leg and the foot, the ankle angle α being zero in a standing position where the lower leg is perpendicular to the foot.
**Figure 3** is a representation of the device 100 according to a bistable embodiment of the invention.
**Figure 4** is a representation of the configurations of the device 100 in a bistable embodiment as a function of the gait stage.
**Figure 5** is a representation of the device 100 according to a monostable embodiment of the invention.
**Figure 6** is a representation of the configurations of the device 100 in a monostable embodiment as a function of the gait stage.
**Figure 7** is a view of the system 200 as a complete ankle prosthesis.
**Figure 8** is a schematic side view of the device 100 in the ankle prosthesis of Figure 7.
**Figure 9** is a representation of the device 100 according to a bistable embodiment of the invention comprising two pairs of rollers.
**Figure 10** is a composite graph comparing the efficiency of an active prosthesis according to the invention and a passive configuration according to the prior art. **Figure 10A** represents the torque T (in Nm/kg) generated by the prostheses during the gait cycle (in % of progress of gait cycle). **Figure 10B** represents the torque T (in Nm/kg) generated by the prostheses as a function of the ankle angle α (in degrees). **Figure 10C** represents the mechanical energy E (in J/kg) accumulated in the prostheses during a gait cycle (in % of progress of gait cycle).

### EXAMPLE

### Example 1: prosthesis for an ankle joint with a bistable embodiment of the priming system.

The present invention is further illustrated by the following example of a prosthesis for an ankle joint embedding this locking mechanism in its bistable configuration.

In this example, the system 200 is a prosthesis for an ankle joint as represented in figures 7 and 8. In this embodiment, the curvilinear axis z is longitudinal and linear. The forward direction (towards front side 102a) is in the walking direction.

The device 100 comprises one elastic element 104 which is a compression spring configured to be compressed by a force exerted towards the rear side 102b (*i.e.,* towards the heel). The priming system 107 comprises one engaging system 107a which is an electromagnet fixed to the support 101, one plunger 107b which is a magnet fixed to the unidirectional locking system 105 and two secondary elastic elements 107c. The two secondary elastic elements 107c are compression springs configured to be compressed by a force exerted towards the front side 102a (*i.e.,* towards the toes). The carriage 103 comprises a cavity 103a having a section decreasing in the backward direction. The unidirectional locking system 105 comprises two rollers 105a disposed in the carriage 103 on each side of the slider 106.

Figure 4 represents the configurations of the device 100 as a function of the stage of the gait cycle.

Figure 4a represents the end of the swing phase. At this phase, the electromagnet is powered off and the unidirectional locking system 105 is abutting against the support 101 thanks of the attraction between the magnet fixed to the unidirectional locking system 105 and the magnetic circuit of the electromagnet fixed to the support 101. The positive ankle angle α implies that the slider 106 is in the rear side of the carriage 103.

Before the heel strike, the unidirectional locking system 105 is primed by the power on of the electromagnet. The secondary elastic elements 107c slightly push the unidirectional locking system 105 towards the rear side of the carriage 103 as pointed by the large grey arrow of figure 4b. At this phase, the ankle angle α between the sole of the foot and the leg decreases thereby leading to plantarflexion. The slider 106 is thus moving in the forward direction in the carriage 103.

At figure 4c, the maximum plantarflexion (MP) is reached. The slider 106 has moved in the forward direction until its maximum position (as pointed by the by the large grey arrow). The ankle angle α between the sole of the foot and the leg reverts and increases leading to dorsiflexion.

The body forward progression of the user creates a force, transmitted by the transmission element 204, on the slider 106 towards the rear side 102b. However, the unidirectional locking system 105 being primed, the slide of the slider 106 towards the backward direction is prevented and the force applied to the slider 106 is thus totally transmitted to the carriage 103 which moves in the backward direction (figure 4d). The movement of the carriage 103 compresses the elastic element 104 which stores the mechanical energy generated by the applied force (as pointed by the by the large grey arrow). The storing of the energy is performed until maximum dorsiflexion (MD) is reached.

During the push-off (slightly before the toe-off), the stored energy is released until the toes are not contacting the floor anymore. This provides the necessary power to the person to push its weight forwards so that the swing phase is initiated.

In figure 4e representing a part of the swing phase, the actuator 110 pushes the slider 106 in the forward direction so that the unidirectional locking system 105 abuts against the support 101 (as pointed by the by the large grey arrow). The electromagnet and the magnet are in contact (first state) rendering the device 100 ready for the next step. Then, the foot completes its return motion.

Compared to known ankle prosthesis, the device 100 of the invention is more compact and more robust thanks to the low number of mechanical elements.

Moreover, the absence of gear wheel allows to increase the comfort for the user.

Finally, since energy is only required to switch from the first to the second state, the autonomy of the device 100 is also increased allowing the user to walk for long time.

### Example 2: prosthesis for an ankle joint with a monostable embodiment of the priming system.

The present invention is further illustrated by the following example of a prosthesis for an ankle joint embedding this locking mechanism in its monostable configuration.

Contrarily to the example 1, the priming system 107 comprises one engaging system 107a which is a stator coil fixed to the support, one plunger 107b which is a magnet slidably inserted into the stator coil and two secondary elastic elements 107c. The two secondary elastic elements 107c are compression springs configured to be compressed by a force exerted towards the rear side 102b *(i.e.,* towards the heel).

Figure 6 represents the configurations of the device 100 as a function of the stage of the gait cycle.

Figure 6a represents the heel strike HS. At this phase, the stator coil is powered off and the unidirectional locking system 105 is abutting against the support 101 thanks to the force exerted by the secondary elastic elements 107c. The positive ankle angle α implies that the slider 106 is in the rear side of the carriage 103.

Between the heel strike HS and the maximum plantarflexion MP, the unidirectional locking system 105 is primed by the power on of the stator coil. The magnetic flux created by the stator coil pushes the magnet towards the backward direction thereby pushing the unidirectional locking system 105 towards the rear side of the carriage 103 as pointed by the large grey arrow of figure 6b. The secondary elastic elements 107c are compressed thereby storing energy.

After the switch to the second state, the ankle angle α between the sole of the foot and the leg decreases thereby leading to a plantarflexion. The slider 106 is thus moving in the forward direction in the carriage 103 (as represented by the large grey arrow in figure 6c). Therefore, the stator coil needs to remain energized to avoid the unidirectional locking system 105 to be disabled by the energy stored in the secondary elastic elements 107c.

When the maximum plantarflexion MP is reached, the slider 106 has moved in the forward direction until its maximum position. The ankle angle α between the sole of the foot and the leg reverts and increases so that the plantarflexion becomes a dorsiflexion.

In figure 6d, the body forward progression of the user creates a force, transmitted by the transmission element 204, on the slider 106 towards the rear side 102b. However, the unidirectional locking system 105 being primed, the slider 106, the carriage 103 and the rollers 105a are buttressed and the stator coil can be powered off. The slide of the slider 106 towards the backward direction is prevented and the force applied to the slider 106 is thus totally transmitted to the carriage 103 which moves in the backward direction. The movement of the carriage 103 compresses the elastic element 104 which stores the mechanical energy generated by the applied force (large grey arrow in figure 6d). The storing of the energy is performed until maximum dorsiflexion MD is reached.

During the push-off (slightly before the toe-off), the stored energy is released (figure 6e) until the toes are not contacting the floor anymore. This provides the necessary power to the person to push its weight forwards so that the swing phase is initiated. The carriage 103 is moving in the forward direction carrying the slider 160 with it (the slider is thus resting respectively to the carriage 103).

Because of the decreasing ankle angle α, the slider 106 continues to move in the forward direction (as pointed by the by the large grey arrow of figure 6f) and is thus moving in the first direction with respect to the carriage 103. The slider 106, the carriage 103 and the rollers 105a are not buttressed anymore and the energy stored in the secondary elastic elements 107c is released so that the unidirectional locking system 105 abuts against the support 101. The device 100 ready for the next step.

Compared to known ankle prosthesis, the device 100 of the invention is more compact and more robust thanks to the low number of mechanical elements.

Moreover, the absence of gear wheel allows to increase the comfortability for the user.

### Example 3: comparison between an active prosthesis for an ankle joint in the bistable embodiment of the priming system and a passive configuration.

This example compares the performances of the active prosthesis 100 for an ankle joint presented in example 1 (bistable embodiment) with the same prosthesis in a configuration 500 where the spring (elastic element) engages with the joint motion later during dorsiflexion at a fixed joint angle. This joint angle is chosen not to impede the foot return motion during the swing phase (for instance, the actuator - or motor comprised in the actuator - does not need to work against the spring to bring the foot back to its neutral position). This configuration 500 does not require the coupling/locking mechanism (active priming) which is an object of the disclosure. This configuration 500 leads to a degraded mechanical response.

In the active prosthesis 100 in bistable embodiment, the first state is achieved at the maximum plantarflexion, while the second state is actively primed at the end of the swing phase.

In the passive configuration 500, the elastic element for storing mechanical energy is a spring. The spring passively engages after the heel strike HS, when the first body 201 crosses the vertical and the ankle angle α becomes positive. Since the spring is not actively engaged, the accumulation of the mechanical energy in the spring starts later in the gait cycle compared to the active prosthesis of the invention.

The torque T in Newton meter per kilogram (Nm/kg) and the accumulated mechanical energy E in Joule per kilogram (J/kg) are measured for a transfemoral user with a body weight of 78 kg, a height of 1.8 m and Medicare Functional Classification Level (K-level) of K3-K4. During the measurements, the user is walking on a treadmill at his comfortable walking speed.

The evolution of the torque T during the gait cycle (expressed as a percentage) generated by the active prosthesis 100 and the passive configuration 500 is represented in figure 10A. The torque T rises earlier in the active prosthesis 100 thanks to the active priming of the unidirectional locking system 105. This prevents the flat low torque feeling experienced by the user in the passive configuration 500 wherein the prosthesis does not offer much resistance to the motion.

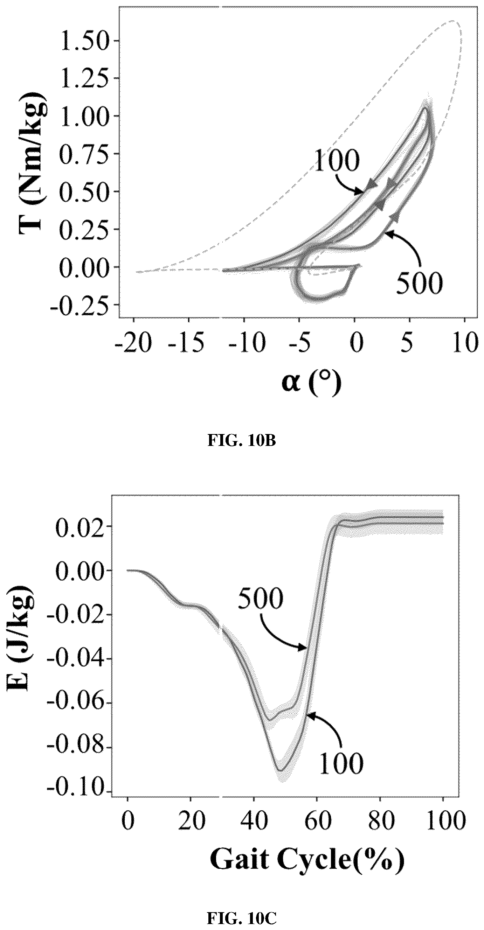

Compared to the reference evolution of the torque experienced in the human ankle joint already presented in figure 2 as a function of the ankle angle α (represented in dashed line), the torque T produced with the active prosthesis 100 increases after the maximum plantarflexion MP and follows a trajectory which is closer to the reference trajectory (figure 10B).

The accumulated mechanical energy E measured during the gait cycle (expressed as a percentage) with the active prosthesis 100 and the passive configuration 500 is represented in figure 10C. The accumulated mechanical energy E for the active prosthesis 100 presents a negative peak with a higher absolute value compared to the passive configuration 500. Therefore, more mechanical energy E is stored in the spring thanks to the active priming of the unidirectional locking system 105.

Finally, the user rated the active prosthesis 100 softer and more comfortable than the passive configuration 500.

### Example 4: comparison between an active prosthesis for an ankle joint in the monostable embodiment of the priming system and a passive configuration.

Example 3 is reproduced with the active prosthesis 100 for an ankle joint in a monostable embodiment.

In the active prosthesis 100 in monostable embodiment, the first state is achieved when loads disappears, while the second state is actively primed after heel strike HS.

The results are similar: the user rated the active prosthesis 100 softer and more comfortable than the passive configuration 500.

## Claims

1. A device (100) for a prosthesis or an orthosis for storing mechanical energy, the device (100) comprising:
- A support (101) comprising a front side (102a) and a rear side (102b) defining a curvilinear axis (z);
- A carriage (103) connected to at least one elastic element (104), each elastic element (104) being configured to store energy and one extremity of each elastic element (104) being connected to the support (101);
- A unidirectional locking system (105);
- A slider (106) slidably inserted into the carriage (103);
- A priming system (107) configured to switch between a first state of the device (100) and a second state of the device (100) and vice-versa;
wherein the carriage (103) is configured to move, the move of the carriage (103) leading to storage of energy by the at least one elastic element (104),
**characterized in that** the slider (106) is configured to slide inside the carriage (103), wherein, in the first state, the unidirectional locking system (105) is disabled,
wherein, in the second state, the unidirectional locking system (105) is primed, the slider (106) is free to slide in a first direction into the carriage (103) and the move of the slider (106) in the reverse direction into the carriage (103) is prevented.

2. The device (100) according to claim **1,** wherein the carriage (103) is configured to move along the curvilinear axis (z), the move of the carriage (103) backwards leading to storage of energy by the at least one elastic element (104), the slider (106) being configured to slide inside the carriage (103) along the curvilinear axis (z).

3. The device (100) according to claim **1** or **2,** wherein, in the first state, the unidirectional locking system (105) is abutted towards the front side of the support (101).

4. The device (100) according to any one of claims **1** to **3,** wherein the priming system (107) comprises at least one plunger (107b) and at least one engaging system (107a) fixed to the support (101) or to the carriage (103), the engaging system (107a) switching the plunger (107b) between a first state of the device (100) and a second state of the device (100).

5. The device (100) according to claim **4,** wherein the at least one engaging system (107a) is an electromagnet and the at least one plunger (107b) is a magnet fixed to the unidirectional locking system (105), the device (100) being in the first state when the electromagnet and the magnet are in contact.

6. The device (100) according to claim **4,** wherein the at least one engaging system (107a) is a stator coil and the at least one plunger (107b) is a magnet slidably inserted into the stator coil.

7. The device according to any one of claims **4** to **6** wherein the priming system (107) further comprises at least one secondary elastic element (107c) and wherein, when the device (100) is in the first state, each elastic element (107c) comprises a first potential energy and when the device (100) is in the second state, each elastic element (107c) comprises a second potential energy smaller than the first potential energy.

8. The device (100) according to any one of claims **2** to **7,** wherein the carriage (103) comprises a cavity (103a) having a section decreasing in the backward direction, the slider (106) and the unidirectional locking system (105) being slidably inserted into the cavity (103a) and wherein, in the first state the unidirectional locking system (105) is in the front side of the carriage (103) and, in the second state, the unidirectional locking system (105) is in the back side of the carriage (103).

9. The device (100) according to any one of claims **1** to **8,** wherein the unidirectional locking system (105) comprises rollers (105a) in a roller cage (105b), and, in the second state:
- the slider (106) and the carriage (103) are in contact with rollers (105a); and
- the slider (106), the rollers (105a) and the carriage (103) are buttressed.

10. The device (100) according to any one of claims **1** to **9** further comprising at least one sensor (108), the signal recorded by the at least one sensor (108) triggering the switch from the first state to the second state.

11. The device (100) according to claim **10** further comprising a controller (109) controlling the priming system (107) according to the signal recorded by the sensor (108).

12. The device (100) according to any one of claims **2** to **11** further comprising an actuator (110) configured to actively slide the slider (106) towards the front side (102a) or the rear side (102b) of the support (101).

13. The device (100) according to claim **12,** further comprising a ballscrew (111) allowing to move the slider (106) along the curvilinear direction (z), wherein the curvilinear direction (z) is longitudinal; and wherein the actuator (110) comprises a motor (110a) dragging a belt-pulley transmission (110b) thereby rotating the ballscrew (111).

## Patentansprüche

1. Vorrichtung (100) für eine Prothese oder eine Orthese zum Speichern von mechanischer Energie, wobei die Vorrichtung (100) Folgendes umfasst:
- eine Stütze (101), die eine Vorderseite (102a) und eine Hinterseite (102b) umfasst, die eine kurvenförmige Achse (z) definiert;
- einen Schlitten (103), der mit mindestens einem elastischen Element (104) verbunden ist, wobei jedes elastische Element (104) so konfiguriert ist, dass es Energie speichert und ein Ende jedes elastischen Elements (104) mit der Stütze (101) verbunden ist;
- ein unidirektionales Verriegelungssystem (105);
- einen Schieber (106), der gleitend in den Schlitten (103) eingesetzt ist;
- ein Ansaugsystem (107), das so konfiguriert ist, dass es zwischen einem ersten Zustand der Vorrichtung (100) und einem zweiten Zustand der Vorrichtung (100) und umgekehrt umschaltet;
wobei der Schlitten (103) so konfiguriert ist, dass er sich bewegt, wobei die Bewegung des Schlittens (103) zum Speichern von Energie durch das mindestens eine elastische Element (104) führt,
**dadurch gekennzeichnet, dass** der Schieber (106) so konfiguriert ist, dass er innerhalb des Schlittens (103) gleitet,
wobei in dem ersten Zustand das unidirektionale Verriegelungssystem (105) deaktiviert ist,
wobei in dem zweiten Zustand das unidirektionale Verriegelungssystem (105) aktiviert ist, der Schieber (106) in einer ersten Richtung in den Schlitten (103) frei gleiten kann und die Bewegung des Schiebers (106) in die entgegengesetzte Richtung in den Schlitten (103) verhindert ist.

2. Vorrichtung (100) nach Anspruch **1,** wobei der Schlitten (103) so konfiguriert ist, dass er sich entlang der kurvenförmigen Achse (z) bewegt, wobei die Bewegung des Schlittens (103) nach hinten zum Speichern von Energie durch das mindestens eine elastische Element (104) führt, wobei der Schieber (106) so konfiguriert ist, dass er innerhalb des Schlittens (103) entlang der kurvenförmigen Achse (z) gleitet.

3. Vorrichtung (100) nach Anspruch **1** oder **2,** wobei in dem ersten Zustand das unidirektionale Verriegelungssystem (105) an der Vorderseite der Stütze (101) anliegt.

4. Vorrichtung (100) nach einem der Ansprüche **1** bis **3,** wobei das Ansaugsystem (107) mindestens einen Kolben (107b) und mindestens ein an der Stütze (101) oder an dem Schlitten (103) befestigtes Kupplungssystem (107a) umfasst, wobei das Kupplungssystem (107a) den Kolben (107b) zwischen einem ersten Zustand der Vorrichtung (100) und einem zweiten Zustand der Vorrichtung (100) umschaltet.

5. Vorrichtung (100) nach Anspruch **4,** wobei das mindestens eine Kupplungssystem (107a) ein Elektromagnet ist und der mindestens eine Kolben (107b) ein Magnet ist, der an dem unidirektionalen Verriegelungssystem (105) befestigt ist, wobei sich die Vorrichtung (100) in dem ersten Zustand befindet, in dem der Elektromagnet und der Magnet in Kontakt sind.

6. Vorrichtung (100) nach Anspruch **4,** wobei das mindestens eine Kupplungssystem (107a) eine Statorspule ist und der mindestens eine Kolben (107b) ein Magnet ist, der gleitend in die Statorspule eingesetzt ist.

7. Vorrichtung nach einem der Ansprüche **4** bis **6,** wobei das Ansaugsystem (107) ferner mindestens ein sekundäres elastisches Element (107c) umfasst und wobei, wenn sich die Vorrichtung (100) in dem ersten Zustand befindet, jedes elastische Element (107c) eine erste potenzielle Energie umfasst und wenn sich die Vorrichtung (100) in dem zweiten Zustand befindet, jedes elastische Element (107c) eine zweite potenzielle Energie umfasst, die kleiner ist als die erste potenzielle Energie.

8. Vorrichtung (100) nach einem der Ansprüche **2** bis **7,** wobei der Schlitten (103) einen Hohlraum (103a) umfasst, der einen Querschnitt aufweist, der sich in Rückwärtsrichtung verjüngt aufweist, wobei der Schieber (106) und das unidirektionale Verriegelungssystem (105) gleitend in den Hohlraum (103a) eingeführt sind und wobei sich in dem ersten Zustand das unidirektionale Verriegelungssystem (105) an der Vorderseite des Schlittens (103) befindet und sich in dem zweiten Zustand das unidirektionale Verriegelungssystem (105) auf der Rückseite des Schlittens (103) befindet.

9. Vorrichtung (100) nach einem der Ansprüche **1** bis **8,** wobei das unidirektionale Verriegelungssystem (105) Rollen (105a) in einem Rollenkäfig (105b) umfasst, und in dem zweiten Zustand:
- der Schieber (106) und der Schlitten (103) mit Rollen (105a) in Kontakt sind; und
- der Schieber (106), die Rollen (105a) und der Schlitten (103) verkeilt sind.

10. Vorrichtung (100) nach einem der Ansprüche **1** bis **9,** ferner umfassend mindestens einen Sensor (108), wobei das von dem mindestens einen Sensor (108) erfasste Signal den Wechsel von dem ersten Zustand in den zweiten Zustand auslöst.

11. Vorrichtung (100) nach Anspruch **10,** die ferner eine Steuerung (109) umfasst, die das Ansaugsystem (107) gemäß dem von dem Sensor (108) aufgezeichneten Signal steuert.

12. Vorrichtung (100) nach einem der Ansprüche **2** bis **11,** ferner umfassend einen Aktuator (110), der so konfiguriert ist, dass er den Schieber (106) aktiv in Richtung der Vorderseite (102a) oder der Rückseite (102b) der Stütze (101) verschiebt.

13. Vorrichtung (100) nach Anspruch **12,** ferner umfassend eine Kugelumlaufspindel (111), die es ermöglicht, den Schieber (106) entlang der kurvenförmigen Richtung (z) zu bewegen, wobei die kurvenförmige Richtung (z) längs ist; und wobei der Aktuator (110) einen Motor (110a) umfasst, der ein Riemen-Riemenscheibengetriebe (110b) antreibt und dadurch die Kugelumlaufspindel (111) dreht.

## Revendications

1. Un dispositif (100) pour une prothèse ou une orthèse destiné au stockage d'énergie mécanique, le dispositif (100) comprenant :
- un support (101) comprenant un côté avant (102a) et un côté arrière (102b) définissant un axe curviligne (z) ;
- un chariot (103) relié à au moins un élément élastique (104), chaque élément élastique (104) étant configuré pour stocker de l'énergie et une extrémité de chaque élément élastique (104) étant reliée au support (101) ;
- un système de verrouillage unidirectionnel (105) ;
- un élément coulissant (106) inséré de manière coulissante dans le chariot (103) ;
- un système d'amorçage (107) configuré pour commuter entre un premier état du dispositif (100) et un second état du dispositif (100), et inversement ;
dans lequel le chariot (103) est configuré pour se déplacer, le déplacement du chariot (103) conduisant au stockage d'énergie par ledit au moins un élément élastique (104),
**caractérisé en ce que** l'élément coulissant (106) est configuré pour coulisser à l'intérieur du chariot (103),
dans lequel, dans le premier état, le système de verrouillage unidirectionnel (105) est désactivé,
et dans lequel, dans le second état, le système de verrouillage unidirectionnel (105) est armé, l'élément coulissant (106) est libre de coulisser dans une première direction à l'intérieur du chariot (103) et le déplacement de l'élément coulissant (106) dans la direction inverse à l'intérieur du chariot (103) est empêché.

2. Le dispositif (100) selon la revendication 1, dans lequel le chariot (103) est configuré pour se déplacer le long de l'axe curviligne (z), le déplacement du chariot (103) vers l'arrière conduisant au stockage d'énergie par ledit au moins un élément élastique (104), l'élément coulissant (106) étant configuré pour coulisser à l'intérieur du chariot (103) le long de l'axe curviligne (z).

3. Le dispositif (100) selon la revendication 1 ou 2, dans lequel, dans le premier état, le système de verrouillage unidirectionnel (105) est en butée vers le côté avant du support (101).

4. Le dispositif (100) selon l'une quelconque des revendications 1 à 3, dans lequel le système d'amorçage (107) comprend au moins un piston (107b) et au moins un système d'engagement (107a) fixé au support (101) ou au chariot (103), le système d'engagement (107a) commutant le piston (107b) entre le premier état du dispositif (100) et le second état du dispositif (100).

5. Le dispositif (100) selon la revendication 4, dans lequel ledit au moins un système d'engagement (107a) est un électroaimant et ledit au moins un piston (107b) est un aimant fixé au système de verrouillage unidirectionnel (105), le dispositif (100) étant dans le premier état lorsque l'électroaimant et l'aimant sont en contact.

6. Le dispositif (100) selon la revendication 4, dans lequel ledit au moins un système d'engagement (107a) est une bobine de stator et ledit au moins un plongeur (107b) est un aimant inséré de manière coulissante dans la bobine de stator.

7. Le dispositif selon l'une quelconque des revendications 4 à 6, dans lequel le système d'amorçage (107) comprend en outre au moins un élément élastique secondaire (107c), et dans lequel, lorsque le dispositif (100) est dans le premier état, chaque élément élastique (107c) présente une première énergie potentielle et lorsque le dispositif (100) est dans le second état, chaque élément élastique (107c) présente une seconde énergie potentielle inférieure à la première énergie potentielle.

8. Le dispositif (100) selon l'une quelconque des revendications 2 à 7, dans lequel le chariot (103) comprend une cavité (103a) présentant une section décroissante dans la direction arrière, l'élément coulissant (106) et le système de verrouillage unidirectionnel (105) étant insérés de manière coulissante dans la cavité (103a), et dans lequel, dans le premier état, le système de verrouillage unidirectionnel (105) est situé du côté avant du chariot (103) et, dans le second état, le système de verrouillage unidirectionnel (105) est situé du côté arrière du chariot (103).

9. Le dispositif (100) selon l'une quelconque des revendications 1 à 8, dans lequel le système de verrouillage unidirectionnel (105) comprend des rouleaux (105a) dans une cage à rouleaux (105b), et, dans le second état :
- l'élément coulissant (106) et le chariot (103) sont en contact avec les rouleaux (105a) ; et
- l'élément coulissant (106), les rouleaux (105a) et le chariot (103) sont en butée.

10. Le dispositif (100) selon l'une quelconque des revendications 1 à 9, comprenant en outre au moins un capteur (108), le signal enregistré par ledit au moins un capteur (108) déclenchant le passage du premier état au second état.

11. Le dispositif (100) selon la revendication 10, comprenant en outre un contrôleur (109) commandant le système d'amorçage (107) en fonction du signal enregistré par le capteur (108).

12. Le dispositif (100) selon l'une quelconque des revendications 2 à 11, comprenant en outre un actionneur (110) configuré pour faire coulisser activement l'élément coulissant (106) vers le côté avant (102a) ou le côté arrière (102b) du support (101).

13. Le dispositif (100) selon la revendication 12, comprenant en outre une vis à billes (111) permettant de déplacer l'élément coulissant (106) le long de la direction curviligne (z), dans lequel la direction curviligne (z) est longitudinale, et dans lequel l'actionneur (110) comprend un moteur (110a) entraînant une transmission par courroie-poulies (110b), entraînant ainsi la rotation de la vis à billes (111).
